# EUROPEAN PATENT SPECIFICATION

(11) **EP 1 963 270 B1**
(45) Date of publication and mention of the grant of the patent: **05.08.2009**
(21) Application number: 06804891.7
(22) Date of filing: 29.11.2006
(51) Int. Cl.: C07D 213/82, A61K 31/435, A61P 29/00

(54) **QUATERNARY 3 -AMIDO, N-METHYLPYRIDINIUM SALTS AS ANTI -INFLAMMATORY AGENTS**
QUATERNÄRE 3-AMIDO, N-METHYLPYRIDINIUMSALZE ALS ENTZÜNDUNGSHEMMENDE MITTEL
SELS QUATERNAIRES DE 3-AMIDO, N-METHYLPYRIDINIUM EN TANT QU'AGENTS ANTI-INFLAMMATOIRES

(30) Priority: 09.12.2005 CH 19542005
(43) Date of publication of application: 03.09.2008
(73) Proprietor: Cerecon AG, 4416 Budendorf (CH)
(72) Inventor: GOSTELI, Jacques, CH-4059 Basel (CH)
(74) Representative: Arnold, Winfried
(86) International application number: PCT/CH2006/000672
(87) International publication number: WO 2007/065281

(56) References cited:
- WO-A-00/40559
- WO-A-20/05067927
- DE-A1- 3 340 028

## Description

### Field of the Invention

The present invention relates to new compounds of anti-inflammatory activity, a process for their production, pharmaceutical compositions comprising the compounds and methods of use thereof. In particular the compounds are acid addition salts composed of the anion of a non-steroidal anti-inflammatory drug (NSAID) and the quaternary cation of N-methylnicotinamide (NMN).

### State of the Art

Salts of NSAID are known in the art. These compounds are also in use as antipyretics and analgesics, mostly in their acid state, but preferably as salts, if the salt form provides advantages over the acid. The salts in use heretofore, e.g. described in DE 3336047 (Ciba-Geigy, 1982), are mainly salts of alkali metals, aluminium and zinc. Among the bases of organic nature, representing cations in their protonated form, the following have been used in commercial preparations: d,l-lysine (Aspegic®), diethylamine (Voltaren-Emugel®) or N-hydroxyethylpysolidine (diclofenac N-hydroxyethylpyrrolidine, e. g. Gallacchi G, Marcolongo R, Drugs Exp. Clin. Res., 1993; 19(3): 95-7. While lysine is a naturally occurring physiologically active amino acid, the other two bases are non-physiological compounds. However, in contrast to the salts of the present invention, in none of the enumerated salt cases, be it a metal salt or one of an organic base, contributes the cation intrinsically to the desired antiinflammatory activity.

The anti-inflammatory acivity of nicotinamide (vitamin PP or B3) has been known for some time (Shalita AR et al., Int. J. Dermatol. 1995, vol.34, 434; Namazi MR, FASEB J. 2003, vol.17,1377). Salts of nicotinamide with NSAIDs are not known. Owing to the low basicity of nicotinamide, they would not be stable and readily decompose.

Also the N-methylnicotinamide cation (NMN, formula (I)) 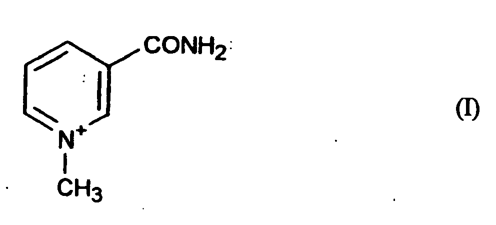 and its activities are known (CAS-Nr. 3106-60-3). NMN is a metabolite of nicotinamide formed in the organism by enzyme-catalyzed N(1)-methylation of nicotinamide. It is excreted as such or oxidized to a pyridone compound prior to elimination from the body. In experiments with rats, NMN exhibits low toxicity, comparable to nicotinamide. In particular, the toxicity of NMN is lower than the one of diethylamine used in commercial salt preparations (Brazda FG, Coulson RA, Proc. Soc. Exptl. Biol. Med. 1946, vol.62, 19).

The anti-inflammatory activity of salts of NMN with certain acids was recently demonstrated in inflammatory skin conditions. Gebicki J. et al. [Polish J. of Pharmacology 2003, vol.55, 109; EP 1 147 086, (2000), and PCT WO 00/40 559, (2000)] showed this activity was clearly more pronounced than the one of nicotinamide. Gebicki et al. in WO 2005/067927 A2 (PHARMENA Sp z.o.o.[PL], 2005-07-28), discloses the salicylate salt of N-methylnicotinamide used to treat inflammatory states. Salts of NMN with NSAIDs have not been described so far.

### Object of the Invention

Theoretically, the possibility would exist of combining the anti-inflammatory activity of the nicotinamide base with that of a nonsteroidal anti-inflammatory active acid by salt formation, thereby expressing the desired activity in one and the same molecule. However, owing to the low basicity of nicotinamide, such combinations would fail: the corresponding salts would decompose by reverse dissociation too readily into the components.

Thus, chemical instability of the acid or improved physiological or pharmacological properties of the salt form, partition coefficient, pH-adjustment, membrane penetration etc. exemplify reasons for searching for a better and improved salt form.

Accordingly, it is an object of the invention to provide novel compounds with an improved anti-inflammatory activity compared to compounds known in the prior art and to avoid certain disadvantages thereof.

It is a further object of the invention to provide a process for the production of the novel compounds.

It is a further object of the invention to provide improved pharmaceutical compositions comprising the novel compounds.

It is a further object of the invention to provide improved methods of use of the novel compounds.

### Detailed Description of the Invention

The invention concerns in the first aspect a compound of the formula (II) 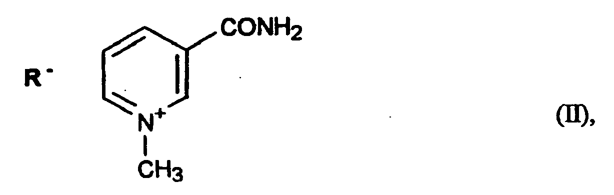 wherein R⁻ is the anion of an acid selected from the group consisting of indomethacin, naproxen, diclofenac, flufenamic acid, mefenamic acid, ibuprofen, etodolac, and lumiracoxib.

The cation of the compound of formula (II), N(1)-methylnicotinamide, is represented by the formula (I) hereinbefore. In formula (II), the groups R⁻ represent anions of a nonsteroidal antiinflammatory Brönsted-acid, e.g. a carboxylic acid belonging to the named group of NSAIDs. R⁻ is preferably the anion of diclofenac or naproxen.

Compounds of the formula (II) have not been described or suggested up to now. They exhibit several advantages in comparison to the known non-steroidal anti-inflammatory drug substances (NSAIDs).

In addition to their double action, the novel salts exhibit surprisingly many advantages over known products. Because of the physiological nature of NMN they are less toxic than salts of nonphysiological bases such as diethylamine. Further, they cannot undergo nitrosation like secondary amines to produce carcinogenic nitrosamines. Furthermore, due to the ionic character of NMN, they are not volatile, imparting therefore higher thermal stability and shelf-life to final products. Reverse dissociation into free base and free NSAID is not possible. Hence, excess of the cationic part (base) is not necessary, as is the case with e.g. diethylamine. In addition, the NMN cation is odourless in contrast to the common organic bases. Aroma chemicals to cover up the evil smelling odour of organic bases, can be omitted in the present final formulations.

In another aspect the invention relates to a process for the preparation of a compound of the formula (II). The process comprises double salt decomposition starting from an NMN-halide and a NSAID or an alkali salt thereof. The preparation of a NMN-salt according to the present invention follows analogous processes known in the art. The process proceeds most readily by double salt decomposition starting from a NMN-halide and a NSAID or a salt thereof, e.g. an alkali salt. Corresponding examples with other acids have been published, e.g. by Sakaki T et al., Biochem. Biophys. Res. Comm. 1978, vol.83, 21 and Ash RP et al., J. Am. Chem. Soc. 1977, vol.99, 4471. The double salt decomposition is carried out in a solvent, in which both starting materials are soluble, e.g. in a lower alcohol, in particular ethanol. The solvent is distilled off, the residue taken up in another less polar solvent, e.g. 1-butanol, and the unsoluble material, e. g. sodium chloride, is filtered off. From the filtrate the desired salt is isolated by evaporation and recristallisation from a suitable solvent mix, such as 1-butanol and an ether, e.g. methyl-t.butylether or diethyl ether.

In another aspect the invention relates to a pharmaceutical preparation comprising a compound according to formula (II). The pharmaceutical formulations are for example of conventional manner. They comprise a compound of formula (II) together with one or more pharmaceutically acceptable carriers, diluents or excipients.

In the treatment of inflammatory conditions or as analgesic, a compound of formula (II) may be administered orally or parenterally to achieve the therapeutic effect in any of the usual pharmaceutical forms. These include solid and liquid unit oral dosage forms such as tablets, capsules, powders, suspensions, solutions and syrups, transdermal plasters, inhalable formulations, and the like, including sustained release preparations, and fluid injectable forms, such as sterile solutions and suspensions. The term dosage form as used in this specification and the claims refer to physically discrete units to be administered in single or multiple dosage to humans or warm-blooded animals, each unit containing a predetermined quantity of active material in association with the required diluent, carrier or vehicle. The quantity of active material is that calculated to produce the desired therapeutic effect upon administration of one or more of such units.

Powders are prepared by comminuting the compound to a suitably fine size and mixing with a similarly comminuted diluent pharmaceutical carrier, such as an edible carbohydrate material as for example, starch. Sweetening, flavoring, preservative, dispersing and colouring agents can also be added. Powders are advantageously applied by inhaling and are for this purpose filled into inhalers. Such inhalers for dry powders are known in the art.

Capsules are made by preparing a powder as described above and filling formed gelatin sheaths. A lubricant, such as talc, magnesium stearate and calcium stearate can be added to the powder mixture as an adjuvant before the filling operation. A glidant such as colloidal silica may be added to improve flow properties. A disintegrating or solubilizing agent may be added to improve the availability of the medicament when the capsule is ingested.

Tablets are made by preparing a powder mixture, granulating or slugging, adding a lubricant and disintegrant and pressing into the desired form. A powder mixture is prepared by mixing the compound, suitably comminuted, with a diluent or base such as starch, sucrose, kaolin, dicalcium phosphate and the like. The powder mixture can be granulated by wetting with a binder such as syrup, starch paste, acacia mucilage or solutions of cellulosic or polymeric materials and forcing through a screen. As an alternative to granulating, the powder mixture can be run through the tablet machine and the resulting imperfectly formed slugs broken into granules. The granules can be lubricated to prevent sticking to the tablet forming dies by means of the addition of stearic acid, a stearate salt, talc or mineral oil. The lubricated mixture is then pressed into tablets. The medicaments can also be combined with free flowing inert carriers and compressed into tablets directly without going through the granulating or slugging steps. A protective coating consisting of a sealing coat of shellac, a coating of sugar or polymeric material and polish coating of wax can be provided. The coating can be resistant in the stomach and the active ingredients to be released in the intestine. Dyestuffs can be added to these coatings to distinguish different unit dosages.

Oral fluids such as syrups and elixirs can be prepared in unit dosage form so that a given quantity, e.g. a teaspoonful, contains a predetermined amount of the compound. Syrups can be prepared by dissolving the active compound in a suitably flavored aqueous sucrose solution, while elixirs are prepared by using a non-toxic alcoholic, e.g. ethanolic, vehicle. Suspensions and emulsions can be formulated by dispersing the medicament in a non-toxic vehicle.

For parenteral administration, fluid unit dosage forms can be prepared by suspending or dissolving a measured amount of the active material in a non-toxic liquid vehicle suitable for injection such as an aqueous, alcoholic, e.g. ethanolic, or oleaginous medium. Such fluid dosage unit forms may contain solubilizers, such as a polyethyleneglycol, stabilizers, and buffers, such as a citric acid/sodium citrate buffer, to provide the desired osmotic pressure. Alternatively, a measured amount of the active material is placed in a vial and the vial and its contents are sterilized and sealed. An accompanying vial or vehicle can be provided for mixing prior to administration. Solutions can also be specifically prepared for inhalation and applied by means of an inhaler. Inhalers for fluids are known in the art.

For transdermal application powders or syrups may be manufactured into suitable transdermal plasters. Such plasters are known in the art.

Of particular utility for the treatment of skin conditions are topical formulations. The type of carrier utilized in the present invention depends on the type of product form desired for the composition. The topical compositions may be a wide variety of product forms for example as are known in the art. These include lotions, creams, emulsions, gels, sticks, shampoos, soaps, sprays, ointments, and pastes. These product forms may comprise several types of carriers including solutions, aerosols, emulsions, gels, solids, and liposomes. Topical formulations are most suitably in the form of an ointment, emulsion gel, gel, cream, shampoo, soap, spray, lotion or a solution.

The compound of formula (II) may be administered topically to the skin (including the scalp), or to the mucosal surfaces, for example by intranasal, oral, intravaginal or intrarectal administration. Preferred is topical administration to the skin at the location of the principal manifestation of the skin disease or disorder, the burn or wound.

The topical formulations of the present invention comprise a safe and effective amount of a dermatologically acceptable carrier within which the compound of formula (II) and other optional components are incorporated to enable the compound of formula (II) and other optional components to be delivered to the skin or other relevant site at an appropriate concentration. The carrier can thus act as a diluent, dispersant, solvent, or the like which ensures that the formulation can be applied to and distributed evenly over the selected target to provide an appropriate concentration of the compound of formula (II).

Preferred topical formulations according to the present invention comprise about 90 to 99.95% of a pharmaceutical base carrier and about 0.005 to about 10% by weight of a compound of formula (II) as defined above. More preferably, the topical formulation contains about 0.01 to about 10 % by weight of a compound of formula (II). Preferred pharmaceutical base carriers are an ointment, emulsion gel, or aqueous solution. In an ointment the compound of formula (II) is preferably present at a concentration by weight of 0.1 to 10%, more preferably 0.5 to 10%. In an emulsion-gel the compound of formula (II) is preferably present in a concentration by weight of 0.05 to 2%, more preferably 0.05 to 1 %, most preferably 0.1 to 0.5%. In a solution, the compound of formula (II) is preferably present in a concentration by weight of 0.1 to 1 %.

The carrier may contain one or more dermatologically acceptable solid, semi-solid or liquid fillers, diluents, solvents, extenders and the like. The carrier may be solid, semi-solid or liquid. Preferred carriers are substantially liquid. The carrier can itself be inert or it can possess dermatological benefits of its own. Concentrations of the carrier can vary with the carrier selected and the intended concentrations of the compound of formula (II) and the other optional components.

Suitable carriers include conventional or otherwise known carriers that are pharmacologically, in particular dermatologically acceptable. The carrier should also be physically and chemically compatible with the compound of formula (II), and should not unduly impair stability, efficacy or other benefits associated with the formulations of the present invention.

Preferred components of the formulations of the present invention should be capable of being commingled in a manner such that there is no interaction, which would substantially reduce the efficacy of the formulation under ordinary use situations.

Preferred carriers contain a dermatologically acceptable, hydrophilic diluent. As used herein, "diluent" includes materials in which the compound of formula (II) can be dispersed, dissolved, or otherwise incorporated. Non limiting examples of hydrophilic diluents are water, organic hydrophilic diluents such as lower monovalent alcohols (e.g., C₁ - C₄) and low molecular weight glycols and polyols, including propylene glycol, polyethylene glycol (e.g., Molecular Weight 200-600 g/mole), polypropylene glycol (e.g. Molecular weight 425-2025 g/mole), glycerol, butylene glycol, 1,2,4-butanetriol, sorbitol esters, 1,2,6-hexanetriol, ethanol, isopropanol, sorbitol esters, butanediol, ethoxylated or propoxylated alcohols and combinations thereof. Water is a preferred diluent. The composition preferably comprises from about 60% to about 99.99% of the hydrophilic diluent.

Solutions according to the subject invention typically include a dermatologically acceptable hydrophilic diluent. Solutions useful in the subject invention preferably contain from about 60% to about 99.99% of the hydrophilic diluent.

Aerosols according to the subject invention can be formed by adding a propellant to a solution such as described above. Exemplary propellants include chloro-fluorinated lower molecular weight hydrocarbons. Additional propellants that are useful herein are described in Sagarin, Cosmetics Science and Technology, 2nd Edition, VoL 2, pp. 443-465 (1972), incorporated herein by reference. Aerosols are typically applied to the skin as a spray-on product. The topical compositions of the subject invention, including but not limited to lotions and creams, may comprise a dermatologically acceptable emollient. Such compositions preferably contain from about 2% to about 50% of the emollient. Emollients tend to lubricate the skin, increase the smoothness and suppleness of the skin, prevent or relieve dryness of the skin, and/or protect the skin. Emollients are typically water-immiscible, oily or waxy materials. A wide variety of suitable emollients are known and may be used herein. Sagarin, Cosmetics Science and Technology, 2nd Edition, Vol. 1, pp. 32-43 (1972), incorporated herein by reference, contains numerous examples of materials suitable as an emollient.

Lotions and creams according to the present invention generally comprise a solution carrier system and one or more emollients. Lotions typically comprise from about 1% to about 20%, preferably from about 5% to about 10%, of emollient; from about 50% to about 90%, preferably from about 60% to about 80%, of water. A cream typically comprises from about 5% to about 50%, preferably from about 10% to about 20%, of emollient; and from about 45% to about 85%, preferably from about 50% to about 75%, of water.

Ointments of the present invention may comprise a simple carrier base of animal or vegetable oils or semi-solid hydrocarbons (oleaginous); absorption ointment bases which absorb water to form emulsions; or water soluble carriers, e.g., a water soluble solution carrier. Ointments may further comprise a thickening agent, such as described in Sagarin, Cosmetics, Science and Technology, 2nd edition, Vol. 1, pp. 72-73 (1972), incorporated herein by reference, and/or an emollient. For example, an ointment may comprise from about 2 % to about 10 % of an emollient; and from about 0.1 % to about 2 % of a thickening agent.

Preferred ointments comprise Eucerine and glycerol; preferred gels comprise methylcellulose, glycerol and water, or comprise polyacrylic acid, polyethylene glycol, ethanol, triethanolamine, paraben and water; preferred solutions comprise aqueous solutions or solutions of ethyl alcohol or propylene glycol.

In another aspect the invention relates to the use of a compound of the formula (II) for the preparation of a pharmaceutical formulation for the treatment of an inflammatory condition in a patient or as analgesic. Such analogous pharmaceutical formulations and their use are known in the art and described hereinbefore.

The following examples demonstrate the processes that can be applied to produce the present salts of the formula (II), the pharmaceutical formulations, and the use thereof. They should not be construed as a limitation thereof.

### Example 1: NMN-salt of Diclofenac

A solution of 3.18 g of diclofenac sodium salt in 25 ml of methanol is mixed with a partial solution of 1.72 g of NMN-chloride in 24 ml of methanol. The resulting clear yellow solution is evaporated to dryness in vacuo to give 4.99 g of yellow foam. This is taken up in 50 ml of 1-butanol, the resulting suspension stirred at 50° C for 15 minutes and the insoluble material filtered off. The filtrate, along with butanol-washings, is again evaporated in vacuo to give 4.42 g of an orange-coloured powder. A solution of this powder in 50 ml of 1-butanol is diluted with three volumes of methyl-t,butylether to precipitate the product. This is collected by suction-filtration, washed with the diluent and dried in vacuo.
Yield: 3.02 g of orange-coloured powder,
m.p. 176-178 °C, C₂₁H₁₉CI₂N₃0₃.

### Example 2: NMN-salt of Naproxen

A solution of 4.00 g of naproxen sodium salt in 40 ml of methanol is combined with a solution of 2.74 g of NMN-chloride in 37 ml of methanol, which solution is prepared in an ultrasound-bath. The resulting yellow solution is condensed to dryness in vacuo. The residue is taken up in 80 ml of 1-butanol, the suspension warmed to 50° C for 15 minutes, the insoluble material filtered off (glass-frit) and washed with butanol (20 ml). Evaporation of the combined filtrate and washings in vacuo yields 6.32 g of crude product. This is dissolved in 80 ml of butanol and precipitated by the addition of 200 ml of ethyl-t.butylether. Filtration and drying of the precipitate gives 4.69 g of orange coloured product. Final purification is achieved by dissolution in 5 ml of hot water, dilution with 20 ml of ethanol and successive precipitation by addition of 150 ml of diethyl ether. The product is collected on a glass frit, washed thrice with diethyl ether and dried in high vacuum.
Yield: 3.52 g of orange-coloured powder
m.p. 99-101° C, C₂₁H₂₂N₂0O₄ x 1.5 H₂O

NMN-halides served as starting materials for the preparation of the salts were first described by Karrer P. ct al., Helv. Chim. Acta 1936, vol.19, 826..

### Example 3: Emulsion-gel

An emulsion-gel is prepared according to DE 333 60 47, Ciba-Geigy 1982. It has the following composition:

| | |
|---|---|
| NMN-salt of diclofenac | 1.36 g |
| Isopropanol | 20.00 g |
| 1,2-Propyleneglycol | 10.00 g |
| Acrylic acid polymerisate (Carbopol 934 P) | 1.20 g |
| Polyhydroxyethylenecetylstearylether(Cetomacrogel 1000) | 0.90 g |
| Paraffinoil, viscous | 2.00 g |
| Capryl/caprinic acid ester (Cetiol LC) | 2.50 g |
| Water, demineralised | ad 100.00 g |

### Preparation of the emulsion-gel:

The NMN-salt of diclofenac (1.36 g) is ground in a chromium-steel mortar. It is then added to the mixture of Carbopol (1.2 g), which is previously mixed with 10 ml of demineralised water, 1,2-propyleneglycol (10.0 g) and, eventually, with another 10 ml of water to form the gel. The paraffin oil (2.0 g) is mixed with Cetomacrogel 1000 (0.9 g) in a chromium-steel dish, Cetiol LC (2.50 g) is added and the mixture molten at 70°C before the whole is added gradually to the vigourously mixed gel containing the active substance. The residual amount of water (to a total of 100 g of water) is gradually added while stirring is being continued for 30 minutes.

### Example 4: Anti-inflammatory Test

The NMN diclofenac salt prepared according to Example 1 was tested in experiments with a trial person in an UV-erythema test according to Hiramatsu Y et al., Arzneimittel-forschung 1990, vol. 40 (11), 1117, and Wilhelm G, Schweiz. Med. Wochenschrift 1949, vol. 79, 577. The criterion of efficacy was the inhibition of the reddening of the skin upon UV-irradiation during the time of observation.

### UV-erythema test (anti-inflammation):

Lamp used: UV C lamp (Sylvania, Hg low pressure), 9 W
Irradiated area: circular, 5 mm in diameter
Distance from lamp: 10 cm
Time of irradiation: 150 seconds
Site: lower arm, shaven, proximal side
Amount of gel applied to irradiated surface: 15 +/-1 mg 3.5 to 5 minutes after end of irradiation

Readings of reddening intensities at given time intervals (arbitrary units):

| Minutes | 40 | 60 | 90 | 120 | 180 (after start of irradiation) |
|---|---|---|---|---|---|
| Untreated | 2 | 2.5 | 3 | 3 | 2.5 |
| NMN-Diclofenac | 1 | 0.5 | 0 | 0.5 | 0.5 |
| Voltaren-Emulgel | 2 | 1.5 | 0.5 | 0.25 | 0.5 |

The test indicate the superior activity of the NMN diclofenac salt over the Voltaren-Emulgel^{®} (diethylammonium salt of diclofenac).

Further similar tests in comparison to the commercially available emulsion gel likewise have shown superiority or at least equal potency.

## Claims

1. A compound of the formula (II) 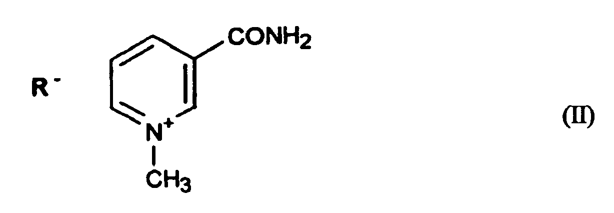 wherein R⁻ is the anion of an acid selected from the group consisting of indomethacin, naproxen, diclofenac, flufenamic acid, mefenamic acid, ibuprofen, etodolac, and lumiracoxib.

2. A compound of the formula (II) according to claim 1, wherein R⁻ is the anion of diclofenac.

3. A compound of the formula (II) according to claim 1, wherein R⁻ is the anion of naproxen.

4. A process for the preparation of a compound of the formula (II) according to anyone of claims 1 to 3 comprising double salt decomposition starting from a halide of N-methylnicotinamide and an anion or an alkali metal salt of an anion of claim 1.

5. The use of a compound of the formula (II) according to anyone of claims 1 to 3 for the preparation of a pharmaceutical formulation for the treatment of an inflammatory condition in a patient.

6. The use of a compound of the formula (II) according to anyone of claims 1 to 3 for the preparation of a pharmaceutical formulation for the treatment of an inflammatory condition in a patient as an analgesic.

7. A pharmaceutical preparation comprising a compound according to anyone of claims 1 to 3 and a pharmaceutical carrier.

## Patentansprüche

1. Eine Verbindung der Formel (II) 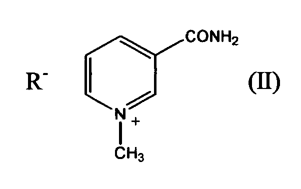 in welcher R⁻ das Anion einer Säure, ausgewählt aus der Gruppe bestehend aus Indomethacin, Naproxen, Diclofenac, Flufenaminsäure, Mefenaminsäure, Ibuprofen, Etodolac und Lumiracoxib bedeutet.

2. Eine Verbindung der Formel (II) gemäss Anspruch 1, in der R⁻ das Anion von Diclofenac bedeutet.

3. Eine Verbindung der Formel (II) gemäss Anspruch 1, in der R⁻ das Anion von Naproxen bedeutet.

4. Ein Verfahren für die Herstellung einer Verbindung der Formel (II) gemäss einem der Ansprüche 1 bis 3, beinhaltend eine doppelte Salzumsetzung ausgehend von einem Halogenid des N(1)-Methylnikotinamids und einem Anion, bzw. einem Alkalimetallsalz eines Anions des Anspruchs 1.

5. Die Verwendung einer Verbindung der Formel (II) gemäss einem der Ansprüche 1 bis 3, für die Herstellung einer pharmazeutischen Formulierung zur Behandlung eines entzündlichen Zustands in einem Patienten.

6. Die Verwendung einer Verbindung der Formel (II) gemäss einem der Ansprüche 1 bis 3, für die Herstellung einer pharmazeutischen Formulierung zur Behandlung eines entzündlichen Zustands eines Patienten als Analgetikum

7. Eine pharmazeutische Präparation enthaltend eine Verbindung gemäss einem der Ansprüche 1 bis 3 und einem pharmazeutischen Trägermaterial.

## Revendications

1. Un composé de la formule (II) 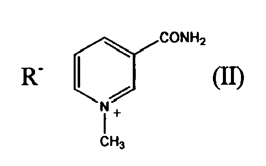 Dans laquelle R signifie l'anion d'une acide sélectionnée du groupe comprenant indomethacin, naproxen, diclofenac, acide flufénamique, acide méfenamique, ibuprofen, etodolac et lumiracoxib.

2. Un composé de la formule (II), selon la revendication 1, dans laquelle R⁻ représente l'anion de diclofenac.

3. Un composé de la formule (II), selon la revendication 1, dans laquelle R⁻ représente l'anion de naproxen.

4. Un procès pour la préparation d'un composé de la formule (II), selon l'une quelconque des revendications 1 à 3, **caracterisé par** une double transformation des sels, à partir d'un halogénure du N(1)-méthylnicotinamide et un anion, par exemple un sel d'alcali d'un anion selon revendication 1.

5. Utilisation d'un composé de la formule (II), selon l'une quelconque des revendications 1 à 3, pour la préparation d'une formulation pharmaceutique pour le traitement d'une condition inflammable d'un patient.

6. Utilisation d'un composé de la formule (II), selon l'une quelconque des revendications 1 à 3, pour la préparation d'une formulation pharmaceutique pour le traitement d'une condition inflammable d'un patient comme analgésique.

7. Une préparation pharmaceutique contenant un composé selon l'une quelconque des revendications 1 à 3 et un matériau porteur.
